## Europäisches Patentamt
## European Patent Office
## Office européen des brevets

(19)

(11) Numéro de publication: **0 233 102**
**A1**

# (12) DEMANDE DE BREVET EUROPEEN

(21) Numéro de dépôt: **87400060.7**

(22) Date de dépôt: **13.01.87**

(51) Int. Cl.⁴: **A 61 F 2/04**

(30) Priorité: **13.01.86 FR 8600464**

(43) Date de publication de la demande: **19.08.87** Bulletin 87/34

(84) Etats contractants désignés: **DE FR GB IT**

(71) Demandeur: **Gaitier, Claude, 13, avenue Maillol, F-95370 Montigny les Corneilles (FR)**

(72) Inventeur: **Gaitier, Claude, 13, avenue Maillol, F-95370 Montigny les Corneilles (FR)**

(74) Mandataire: **Dawidowicz, Armand, 30, Boulevard du Château, F-92200 Neuilly (FR)**

(54) **Oesophage artificiel.**

(57) Prothèse implantable remplissant les fonctions naturelles de l'oesophage, caractérisée en ce qu'elle consiste en un tube à double paroi, à section circulaire dans sa portion médiane et élliptique vers ses extrémités, la paroi extérieure (1) étant réalisée en un matériau synthétique hémocompatible relativement rigide et la paroi intérieure (2) étant réalisée en un matériau synthétique hémocompatible souple, à surface interne parfaitement lisse, une bande hélicoïdale (4) de matériau relativement élastique étant insérée entre les deux parois (1, 2) dans la portion médiane dudit tube.

0233102

OESOPHAGE ARTIFICIEL

La présente invention concerne une nouvelle prothèse, utilisable comme oesophage de remplacement, implantable sur tout sujet atteint d'affections de cet organe.

Les statistiques révèlent que le nombre de ces sujets est en augmentation constante dans le monde, les origines de ces affections pouvant être très diverses:

- affections congénitales
- affections dües à l'ingestion de "produits corrosifs"
- affections d'origine traumatique
- affections ulcéreuses et diverses
- affections dues à des tumeurs malignes.

Très peu de traitements de ces affections sont possibles, mais de toutes façons, il n'existe à ce jour aucune prothèse permettant:

- de prolonger la vie du patient au-delà de 2 ans
- d'éviter la récidive de la tumeur
- d'assurer, en un temps opératoire, une intervention permettant la mise en place d'un implant rétablissant la continuité hypo-pharyngo-gastrique.

La présente invention élimine toutes ces difficultés, grâce à une prothèse de conception complètement nouvelle, de construction et d'implantation simples et remplissant les fonctions naturelles de l'oesophage par transmission des péristaltismes aortique, bronchique et diaphragmique, et permettant l'éradication définitive et en un temps chirurgical de la pathologie initiale pour laquelle l'intervention est pratiquée.

Cette prothèse consiste en un tube à double paroi, à section circulaire dans sa portion médiane et elliptique vers ses extrémités, la paroi extérieure étant réalisée en un matériau synthétique hémocompatible relativement rigide et la paroi intérieure étant réalisée en un matériau synthétique hémocompatible souple, à surface interne parfaitement lisse., une bande hélicoïdale de matériau relativement élastique étant insérée entre les deux parois dans la portion médiane dudit tube.

Dans la mise en pratique de l'invention, le matériau synthétique sera de préférence un polyuréthane, dont la rigidité et la souplesse dépendent du degré de réticulation et de la nature et de la proportion de plastifiant, les diverses qualités de ce matériau et leurs propriétés physiques et mécaniques étant bien connues.

Pour illustrer l'invention, le dessin annexé représente:
- figure 1, l'oesophage artificiel selon l'invention, en section verticale, et
- figure 2 a, 2 b, 2 c, des coupes horizontales à divers niveaux de la prothèse de la figure 1.

Sur ce dessin, on a désigné par la référence 1 la paroi extérieure de la prothèse, et par la référence 2 la paroi intérieure. La matière de la paroi 1 est relativement rigide,bien que suffisamment souple pour réagir au péristaltisme des organes environnants. La paroi pourra avoir une épaisseur de 0,3 à 0,5 mm.

De même la matière de la paroi 2 devra être suffisamment souple pour réagir aux déformations provenant de la paroi 1 de manière à déterminer le transfert du bol alimentaire. Cette paroi 2 aura également une épaisseur de l'ordre de 0,5 à 0,8 mm.

Dans l'espace 3 situé entre les deux parois, d'une épaisseur de l'ordre de 3 mm, est logée une bande hélicoïdale 4, en matériau souple et élastique. La fonction de cette bande est de transmettre élastiquement à la paroi intérieure 2 les déformations subies par la paroi extérieure 1.

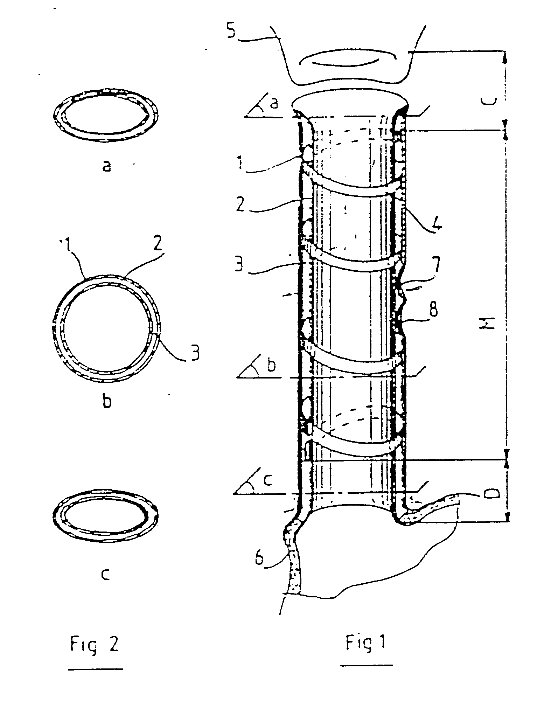

La portion médiane M du tube, d'environ 18-20 cm de long et, comme le montre la figure 2 b, est à section circulaire. Les portions extrêmes, constituant l'oesophage cervical C (d'environ 5 cm) et l'oesophage sous-diaphragmique ( d'environ 3 cm ), sont à section elliptique, comme le montrent les figures 2 a et 2 c respectivement. Ce sont ces sections en forme d'entonnoir qui sont destinées à être raccordées de façon parfaitement étanche respectivement au cartilage cricoïde 5 et à l'estomac 6.

Le fonctionnement de cette prothèse est basé sur les rétrécissements anatomiques et les pressions exercées à ces niveaux par les organes successifs, à savoir:

- la boucle de KILLIAN au niveau de l'entonnoir supérieur C
- le rétrécissement aortique 7
- le rétrécissement bronchique 8 ( dû à la bronche gauche)
- et le rétrécissement diaphragmatique, au niveau de l'entonnoir inférieur D.

Lorsque le bol alimentaire franchit le sphincter cricoïde 5, ce dernier dilate la partie haute C de l'oesophage, et se trouve éjecté dans la portion médiane M, comportant le ressort 4.

Ce ressort transmet à la paroi intérieure 2 les pressions séquentielles exercées sur la paroi extérieure 1 par l'aorte dans la zone 7 puis par la bronche dans la zone 8. Le bol alimentaire progresse ainsi grâce à ce péristaltisme en quelques secondes jusqu'à la portion D de l'oesophage et est éjecté dans l'estomac, la surface lisse de la paroi interne 2 assurant son évacuation totale.

On notera qu'outre la stabilité d'ensemble de là prothèse, la rigidité relative de la paroi 1 évite l'écrasement du tube sur lui-même quand le patient est en position allongée.

L'implantation de cette prothèse se fait selon les techniques chirurgicales usuelles et ne soulève aucun problème particulier. Au contraire, la double paroi du tube facilite une anastomose parfaitement étanche, par exemple au moyen d'un fil non résorbable du type monofil, tout risque de fistule au niveau des points de suture étant évité par un collage terminal.

0233102

La prothèse selon l'invention constitue donc la solution la plus fiable aux diverses affections de l'oesophage, sur lesquels les traitements existants ne pouvaient guère laisser une espérance de survie supérieure à deux ans. Au surplus cette technique élimine les risques de récidive, puisque dans la majorité des cas les cancers de l'oesophage sont pratiquement toujours primitifs.

REVENDICATIONS

1.- Prothèse implantable remplissant les fonctions naturelles de l'oesophage, caractérisée en ce qu'elle consiste en un tube à double paroi, à section circulaire dans sa portion médiane et élliptique vers ses extrémités, la paroi extérieure étant réalisée en un matériau synthétique hémocompatible relativement rigide et la paroi intérieure étant réalisée en un matériau synthétique hémocompatible souple, à surface interne parfaitement lisse, une bande hélicoïdale de matériau relativement élastique étant insérée entre les deux parois dans la portion médiane dudit tube.

2.- Prothèse selon la revendication 1 caractérisée en ce que ledit matériau synthétique est un polyuréthane.

Fig 2

Fig 1

Office européen
des brevets

**RAPPORT DE RECHERCHE EUROPEENNE**

Numéro de la demande

EP 87 40 0060

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | GB-A-2 069 339 (KEYMED)<br>* Page 2, lignes 37-72; figures * | 1 | A 61 F 2/04 |
| | --- | | |
| Y | US-A-4 086 665 (POIRIER)<br>* Abrégé; figures; colonne 1, ligne 67 - colonne 2, ligne 41 * | 1 | |
| | --- | | |
| A | MEDICAL & BIOLOGICAL ENGINEERING & COMPUTING, vol. 23, no. 1, janvier 1985, pages 77-78, IFMBE, Londres, GB; D.P. ORR et al.: "Fabrication techniques for thin-walled, kink-resistant tubular structures for use in medical devices"<br>* Page 77, colonne 1 * | 2 | |
| | --- | | **DOMAINES TECHNIQUES RECHERCHES (Int. Cl.4)** |
| A | US-A-4 130 904 (WHALEN) | | A 61 F<br>F 16 L |
| | ----- | | |

Le présent rapport de recherche a été établi pour toutes les revendications

| Lieu de la recherche | Date d'achèvement de la recherche | Examinateur |
|---|---|---|
| LA HAYE | 03-04-1987 | STEENBAKKER J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82